# EUROPÄISCHE PATENTSCHRIFT

(11) **EP 0 646 362 B1**
(45) Veröffentlichungstag und Bekanntmachung des Hinweises auf die Patenterteilung: **17.11.1999**
(21) Anmeldenummer: 94114804.1
(22) Anmeldetag: 20.09.1994
(51) Int. Cl.: A61C 8/00

(54) **Schraubimplantat**
Screw-type dental implant
Implant à filetage

(30) Priorität: 21.09.1993 DE 4332075
(43) Veröffentlichungstag der Anmeldung: 05.04.1995
(73) Patentinhaber: BEGO Bremer Goldschlägerei Wilh. Herbst GmbH & Co., 28359 Bremen (DE)
(72) Erfinder: Montag, Hans, Dr., D-33615 Bielefeld (DE); Gundlach, Hans-Werner, Dr., D-28209 Bremen (DE)
(74) Vertreter: Möller, Friedrich, Dipl.-Ing.

(56) Entgegenhaltungen:
- EP-A- 0 282 789
- GB-A- 2 199 626
- US-A- 5 000 686

## Beschreibung

Die Erfindung betrifft ein Schraubimplantat zur Befestigung von Zahnersatz am Kiefer gemäß dem Oberbegriff des Anspruchs 1. Ein Schraubimplantat mit den Merkmalen des Oberbegriffs von Anspruch 1 ist aus der US-A-5 000 686 bekannt.

Eingesetzt werden Schraubimplantate zur Befestigung von Zahnersatz, und zwar insbesondere Zahnprothesen und Einzelzähnen, am Kiefer. Die Schraubimplantate werden dazu größtenteils in eine vorgefertigte Aufnahmebohrung im Kiefer eingeschraubt. Bekannte Schraubimplantate dieser Art verfügen insbesondere im Kopfbereich über eine Gestaltung, die beim Einsetzen in die Aufnahmebohrung des Kiefers zu Verquetschungen führt, die ein die Einheilung des Schraubimplantats in dem Kiefer verzögerndes Trauma hervorrufen können. Schließlich bieten die bekannten Schraubimplantate vielfach nur einen unzureichenden Halt im Kiefer.

Hiervon ausgehend liegt der Erfindung die Aufgabe zugrunde, ein Schraubimplantat zu schaffen, das sich leicht in den Kiefer einsetzen läßt und hierin sowohl rasch als auch fest einheilt.

Ein zur Lösung dieser Aufgabe dienendes Schraubimplantat weist die Merkmale des Anspruchs 1 auf. Durch den Mittelabschnitt mit einem Gewinde geringerer Tiefe wird zweierlei erreicht: Zum einen wird am Übergang zum gewindelosen Kopfteil das Zahnfleisch infolge der geringeren Gewindetiefe aufgeweitet bzw. vorgespannt, wodurch es am Mittelabschnitt des Schraubimplantats dichtend anliegt. Während des Einheilprozesses des Schraubimplantats in den Kiefer wird dadurch verhindert, daß Bakterien oder sonstige Verunreinigungen in einen Spalt zwischen dem Schraubimplantat und dem Kiefer bzw. dem Zahnfleisch eindringen. Zum anderen wird durch die verringerte Gewindetiefe der Kerndurchmesser am Mittelabschnitt vergrößert, wodurch die hier zur Verfügung stehende Wandstärke größer wird. Das ermöglicht eine leichtere Unterbringung eines Werkzeugaufnahmemittels, insbesondere eines Innensechskants, im Inneren des Schraubimplantats. Dieser Innensechskant kann sich dann nämlich sowohl über den Kopfabschnitt als auch den Mittelabschnitt erstrecken.

Vorteilhafte Weiterbildungen des Schraubimplantats ergeben sich aus den Unteransprüchen.

Bevorzugte Ausführungsbeispiele der Erfindung werden nachfolgend anhand der Zeichnung näher erläutert. In dieser zeigen:
- Fig. 1: einen stark vergrößerten Längsschnitt durch ein Schraubimplantat,
- Fig. 2: eine Ansicht des Schraubimplantats der Fig. 1 von oben, und
- Fig. 3: eine vergrößerte Einzelheit III aus der Fig. 1.

Die Figuren zeigen in stark vergrößerter Darstellung ein Schraubimplantat 81 zum Eindrehen bzw. Einschrauben in eine vorgefertigte Aufnahmebohrung im Kiefer. Nach dem Einheilen in den Kiefer bildet das Schraubimplantat 81 einen festsitzenden, unverdrehbaren Sockel zum Anschrauben von zum Beispiel Prothesen oder künstlichen (Einzel-)Zähnen.

Das Schraubimplantat 81 verfügt über einen länglichen, zylindrischen Implantatkörper 21. Dieser ist gebildet aus einem nichtrostenden metallischen Material, insbesondere Titan. Ausgehend von einer kieferauswärtsgerichteten Stirnseite 22 ist der Implantatkörper 21 mit einer mittigen Gewindebohrung 23 versehen. Diese erstreckt sich in Richtung einer Längsmittelachse 24 in das Innere des Implantatkörpers 21. Im oberen Endbereich ist die Gewindebohrung 23 mit einer kegelstumpfförmigen Aufweitung versehen, die sich ausgehend von der Stirnseite 22 zur Gewindebohrung 23 hin verjüngt. Die Verjüngung weist bezogen auf die Längsmittelachse 24 einen Winkel auf, der geringfügig kleiner ist als der Winkel eines mit der kegelstumpfförmigen Aufweitung etwa korrespondierenden kegelstumpfförmigen Ansatzes an ein auf das Schraubimplantat 81 aufzusetzendes (nicht gezeigtes) Aufbauteil. Dadurch dichtet das Aufbauteil die kegelstumpfförmige Aufweitung an einer auf der Stirnseite 22 liegenden Kante ab zur Verhinderung des Eindringens von Schmutz oder dergleichen in das Innere des Schraubimplantats 81, insbesondere die Gewindebohrung 23.

Der äußere Mantel des Implantatkörpers 21 ist teilweise mit einem Außengewinde versehen. Der Außenmantel des Schraubimplantats 81 setzt sich nämlich aus drei Abschnitten zusammen, und zwar einem unteren Gewindeabschnitt 82, einem sich daran anschließenden Mittelabschnitt 83 und einem oberen Kopfabschnitt 84. Ein von einer kieferseitigen (unteren) Stirnseite 31 ausgehender Teil des Außengewindes ist als selbstschneidendes Außengewinde 32 ausgebildet. Dieses erstreckt sich etwa über die halbe Länge des Schraubimplantats 81. An das selbstschneidende Außengewinde 32 schließt sich in Richtung zur (oberen) Stirnseite 22 ein reines Schraubgewinde 33 an. Das Außengewinde ist als ein metrisches Gewinde ausgebildet, kann aber auch ein Trapez- oder Rundgewinde sein.

Das untere selbstschneidende Außengewinde 32 ist zum einen gebildet aus dem Schraubgewinde 33, was vom nicht selbstschneidenden oberen Teil des Außengewindes ununterbrochen durchläuft bis zur (unteren) Stirnseite 31. Zum anderen verfügt das selbstschneidende Außengewinde 32 über mehrere Ausnehmungen 34. Es sind im gezeigten Ausführungsbeispiel vier um jeweils 90° zueinander versetzte Ausnehmungen 34 vorhanden. Sie sind gebildet durch Ausfräsungen am Umfang des Implantatkörpers 21 mit rechtwinklig zueinander verlaufenden Flächen. Die radialgerichteten Flächen bilden Schneidflächen 35. Sie liegen auf zwei um 90° zueinander versetzten Längsmittelebenen des Implantatkörpers 21. Rechtwinklig zu den Schneidflächen 35 verlaufende (längere) Flächen bilden Freiflächen 37, die parallel zu der jeweiligen Längsmittelebene verlaufen. Die äußeren Kanten der Schneidflächen 35 bilden Schneidkanten 38. Die auf der jeweiligen Schneidkante 38 liegende Tangente des Umfangs des Implantatkörpers 21 verläuft rechtwinklig zur Schneidfläche 35.

Ein unterer Teilbereich des selbstschneidenden Außengewindes 32 verjüngt sich zur (unteren) Stirnseite 31 des Implantatkörpers 21. Dadurch wird das Einsetzen des Schraubimplantats 81 in die im Kiefer vorbereitete Aufnahmebohrung und das Anschneiden des Gewindes erleichtert.

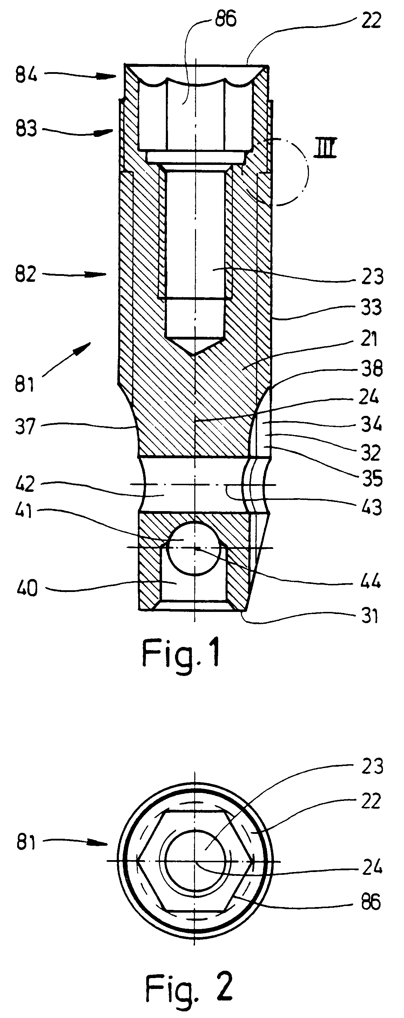

Im Implantatkörper 21 ist eine untere Sackbohrung 40 angeordnet. Diese erstreckt sich ausgehend von der (unteren) Stirnseite 31 des Implantatkörpers 21 in Richtung seiner Längsmittelachse 24. Dem unteren Endbereich der Sackbohrung 40 ist eine quer zur Längsmittelachse 24 verlaufende (erste) Querbohrung 41 zugeordnet. Diese kreuzt die Sackbohrung 40 und steht mit dieser somit in Verbindung (Fig. 1). Die Querbohrung 41 ist als Durchgangsbohrung ausgebildet, derart, daß sie gegenüberliegende Öffnungen im Implantatkörper 21 bildet.

Über der (ersten) Querbohrung 41 ist eine (zweite) Querbohrung 42 angeordnet. Diese ist um 90° zur (ersten) Querbohrung 41 versetzt, so daß sich ihre parallelverlaufenden, horizontalen Längsmittelachsen 43, 44 kreuzen. Die Längsmittelachsen 43 und 44 kreuzen ebenfalls die Längsmittelachse 24 des Implantatkörpers 21 (Fig. 1). Der Abstand der Querbohrungen 41 und 42 ist derart gewählt, daß sie sich nicht schneiden bzw. berühren (Fig. 1).

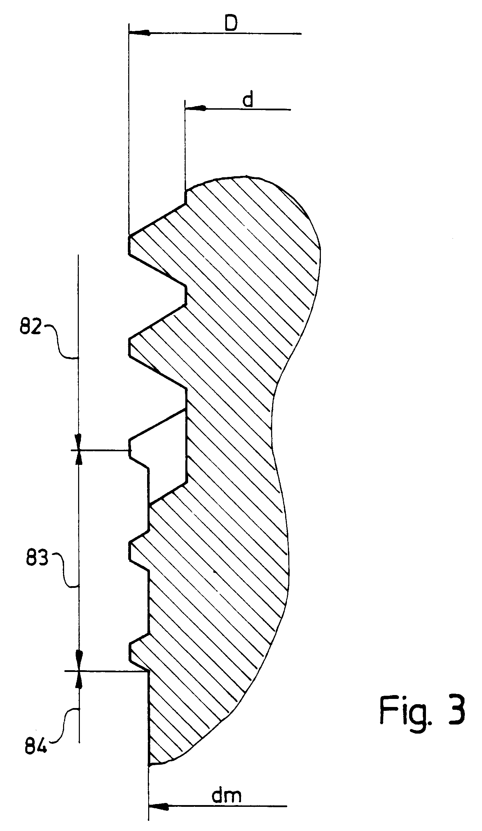

Der Mittelabschnitt 83 verfügt wie der Gewindeabschnitt 82 über ein Außengewinde, wobei das Außengewinde des Gewindeabschnitts 82 kontinuierlich weiterläuft über den gesamten Mittelabschnitt 83. Die Tiefe des Außengewindes ist am Mittelabschnitt 83 jedoch geringer als am Gewindeabschnitt 82. Wie aus der Fig. 3 ersichtlich, ist der (normale) Kerndurchmesser d des Gewindeabschnitts 82 im Bereich des Mittelabschnitts 83 vergrößert. Dadurch verfügt der Mittelabschnitt 83 über einen vergrößerten Kerndurchmesser dm, der etwa zwischen dem Kerndurchmesser d und dem Flankendurchmesser D des regulären Gewindes am Gewindeabschnitt 82 liegt. Der Flankendurchmesser D ist sowohl am Gewindeabschnitt 82 als auch am Mittelabschnitt 83 gleich groß (Fig. 3). Der Übergang zwischen dem Kerndurchmesser d und dem vergrößerten Kerndurchmesser dm kann wie beim gezeigten Ausführungsbeispiel der Fig. 3 von einer Schräge gebildet sein. Es ist aber auch denkbar, den Übergang scharfkantig auszubilden.

Der Kerndurchmesser dm ist über die gesamte Länge des Mittelabschnitts 83 gleich. Es ist aber auch denkbar, daß sich der Kerndurchmesser dm vom Gewindeabschnitt 82 zum Kopfabschnitt 84 hin vergrößert, indem er sich vom normalen Kerndurchmesser d am Gewindeabschnitt 82 erweitert auf den Durchmesser des (glatten) Kopfabschnitts 84.

Aus der Fig. 1 wird erkennbar, daß der vergrößerte Kerndurchmesser dm im Bereich des Mittelabschnitts 83 dem Durchmesser des gewindelosen, vorzugsweise glatten und gegebenenfalls polierten Kopfabschnitts 84 entspricht. Dadurch verfügt der Nutengrund der Rillen des Teilgewindes am Mittelabschnitt 83 über einen Durchmesser, der dem Durchmesser des Kopfabschnitts 84 entspricht.

Das Schraubimplantat 81 verfügt über ein Werkzeugaufnahmemittel, das als ein Innensechskant 86 ausgebildet ist. Der Innensechskant 86 geht aus von der (oberen) Stirnseite 22 des Schraubimplantats 81 und erstreckt sich von hier aus über den gesamten Kopfabschnitt 84 und über den größten Teil des Mittelabschnitts 83. Infolge der vorstehend beschriebenen Verringerung der Tiefe des Außengewindes am Mittelabschnitt 83 wird für den Innensechskant 86 sozusagen Platz geschaffen, weil das den Innensechskant 86 umgebende Material des Schraubimplantats 81 sowohl am Kopfabschnitt 84 als auch am Mittelabschnitt 83 etwa gleich ist.

Das Schraubimplantat 81 kann mit einer Beschichtung versehen sein. Die Beschichtung besteht aus einem bioaktiven oder biokompatiblen Material. Als bioaktives Material kommt insbesondere Hydroxylapatit in Betracht. Beim biokompatiblen Material kann es sich um Titan (Flammenspray) oder TiO₂ handeln. Die zu beschichtenden Flächen sind vor der Beschichtung angerauht, und zwar vorzugsweise durch Strahlen mit Korundmaterial. Die mittlere Rauhigkeit der zu beschichtenden Oberflächen liegt im Bereich von Rₐ2 bis 10µm.

Es kann das gesamte Schraubimplantat 81 beschichtet sein. Es ist aber auch denkbar, von der Beschichtung die innere Gewindebohrung 23 und/oder den Innensechskant 86 auszunehmen. Außerdem kann der gesamte Kopfabschnitt 84 oder ein oberer Teilbereich desselben unbeschichtet sein. Die unbeschichteten Bereiche des Kopfabschnitts 84 verfügen über eine glatte Oberfläche, indem sie vorzugsweise hochglanzpoliert sind.

## Patentansprüche

1. Schraubimplantat zur Befestigung von Zahnersatz am Kiefer, mit einem zumindest teilweise in den Kiefer eindrehbaren Implantatkörper, in dem ein Werkzeugaufnahmemittel (86) zum Einschrauben des Implantats angeordnet ist und der eine Außenfläche aufweist, die an ihrem unteren Teil mindestens teilweise mit einem Außengewinde zur Bildung eines Gewindeabschnitts (82) versehen ist und an einem oberen Teil einen gewindefreien Kopfabschnitt (84) aufweist, wobei zwischen dem Kopfabschnitt (84) und dem Gewindeabschnitt (82) ein Mittelabschnitt (83) mit einem Gewinde geringerer Tiefe angeordnet ist, **dadurch gekennzeichnet, da**ß sich das Werkzeugaufnahmemittel (86) zum Einschrauben des Implantats durch den Kopfabschnitt (84) und mindestens über den größten Teil des Mittelabschnitts (83) mit dem Gewinde geringerer Tiefe erstreckt.

1. Schraubimplantat nach Anspruch 1, dadurch gekennzeichnet, daß der gewindefreie Kopfabschnitt (84) einen kleineren Durchmesser als der Gewindeabschnitt (82) aufweist.

2. Schraubimplantat nach Anspuch 2, dadurch gekennzeichnet, daß der Kerndurchmesser (dm) des Mittelabschnitts (83) etwa dem Durchmesser des Kopfabschnitts (84) entspricht.

3. Schraubimplantat nach einem der vorhergehenden Ansprüche, dadurch gekennzeichnet, daß der Mittelabschnitt (83) länger als der Kopfabschnitt (84) ist.

4. Schraubimplantat nach einem der vorhergehenden Ansprüche, dadurch gekennzeichnet, daß das Werkzeugaufnahmemittel als ein Innensechskant (86) ausgebildet ist.

5. Schraubimplantat nach Anspruch 5, dadurch gekennzeichnet, daß der Innensechskant (86) sich geringfügig unterhalb einer kieferaußenseitigen Stirnseite (22) des Implantatkörpers (21) befindet.

6. Schraubimplantat nach Anspruch 5 oder 6, dadurch gekennzeichnet, daß zwischen der Stirnseite (22) und dem Innensechskant (86) eine kegelstumpfförmige Aufweitung vorhanden ist, die sich in Richtung zum Innensechskant (86) verjüngt.

## Claims

1. Screw-type dental implant for fastening dentures to the jaw, having a dental implant body, which can be screwed at least partly into the jaw and in which a tool-locating means (86) for screwing the dental implant into place is arranged and which has an outer surface which at its bottom part is provided at least partly with an external thread for forming a threaded section (82) and has a thread-free section (84) at a top part, a centre section (83) having a thread of smaller depth being arranged between the head section (84) and the threaded section (82), characterized in that the tool-locating means (86) for screwing the dental implant into place extends through the head section (84) and at least over most of the centre section (83) having the thread of smaller depth.

2. Screw-type dental implant according to Claim 1, characterized in that the thread-free head section (84) has a smaller diameter than the threaded section (82).

3. Screw-type dental implant according to Claim 2, characterized in that the minor diameter (dm) of the centre section (83) corresponds approximately to the diameter of the head section (84).

4. Screw-type dental implant according to one of the preceding claims, characterized in that the centre section (83) is longer than the head section (84).

5. Screw-type dental implant according to one of the preceding claims, characterized in that the tool-locating means is designed as a hexagon socket (86).

6. Screw-type dental implant according to Claim 5, characterized in that the hexagon socket (86) is located slightly below an end face (22), to the outside of the jaw, of the dental implant body (21).

7. Screw-type dental implant according to Claim 5 or 6, characterized in that there is a frustoconical widened portion between the end face (22) and the hexagon socket (86), and this frustoconical widened portion tapers in the direction of the hexagon socket (86).

## Revendications

1. Implant à visser pour la fixation de dents artificielles à la mâchoire, comportant un corps d'implant se vissant au moins en partie dans la mâchoire dans lequel est fait un moyen de réception d'outil (86) pour le vissage de l'implant et qui présente une surface extérieure qui est pourvue dans sa partie inférieure, au moins en partie, d'un filetage extérieur pour la formation d'une partie filetée (82) et présente dans sa partie supérieure une partie de tête sans filetage (84), entre la partie de tête (84) et la partie filetée (82) se trouvant une partie médiane (83) pourvue d'un filetage de moindre profondeur, caractérisé par le fait que le moyen de réception d'outil (86) pour le vissage de l'implant traverse la partie de tête (84) et s'étend au moins sur la plus grande partie de la partie médiane (83) pourvue du filetage de moindre profondeur.

2. Implant à visser selon la revendication 1, caractérisé par le fait que la partie de tête sans filetage (84) a un plus petit diamètre que la partie filetée (82).

3. Implant à visser selon la revendication 2, caractérisé par le fait que le diamètre intérieur (dm) de la partie médiane (83) correspond à peu près au diamètre de la partie de tête (84).

4. Implant à visser selon l'une des revendications précédentes, caractérisé par le fait que la partie médiane (83) est plus longue que la partie de tête (84).

5. Implant à visser selon l'une des revendications précédentes, caractérisé par le fait que le moyen de réception d'outil est un hexagone femelle (86).

6. Implant à visser selon la revendication 5, caractérisé par le fait que l'hexagone femelle (86) se trouve légèrement au-dessous d'une face frontale côté extérieur de la mâchoire (22) du corps d'implant (21).

7. Implant à visser selon l'une des revendications 5 et 6, caractérisé par le fait qu'entre la face frontale (22) et l'hexagone femelle (86) existe un élargissement tronconique qui se rétrécit en direction de l'hexagone femelle (86).
